# FASCICULE DE BREVET EUROPEEN

(11) **EP 3 539 549 B1**
(45) Date de publication et mention de la délivrance du brevet: **28.10.2020**
(21) Numéro de dépôt: 19161666.3
(22) Date de dépôt: 08.03.2019
(51) Int. Cl.: A23K 20/10, A61K 36/48, A61K 36/42, A23K 10/30, A23K 20/163, A23K 50/75, A61P 33/00, A61K 36/82

(54) **ADDITIF ALIMENTAIRE À BASE DE SAPONINES POUR LE TRAITEMENT DE LA COCCIDIOSE**
LEBENSMITTELZUSATZSTOFF AUF DER BASIS VON SAPONINEN FÜR DIE BEHANDLUNG VON KOKZIDIOSE
FOOD ADDITIVE MADE OF SAPONINS FOR THE TREATMENT OF COCCIDIOSIS

(30) Priorité: 12.03.2018 FR 1852100
(43) Date de publication de la demande: 18.09.2019
(73) Titulaire: Nor-Feed, 49070 Beaucouzé (FR)
(72) Inventeur: CHICOTEAU, Pierre, 49240 Avrillé (FR); BERNABIA, Amine, 49100 Angers (FR); WILTZ, Maria, 92160 ANTONY (FR); PREVERAUD, Damien, 03410 Domérat (FR)
(74) Mandataire: Bringer, Mathieu

(56) Documents cités:
- EP-A1- 1 082 909
- WO-A2-2009/138684
- CN-B- 103 478 508
- FR-A1- 2 833 813
- MUTHAMILSELVAN T ET AL: "Herbal remedies for coccidiosis control: A review of plants, compounds, and anticoccidial actions", EVIDENCE-BASED COMPLEMENTARY AND ALTERNATIVE MEDICINE 2016 HINDAWI PUBLISHING CORPORATION USA, vol. 2016, 2016, XP002786334, ISSN: 1741-427X
- DONG Z L ET AL: "The effects of dietary supplementation of pre-microencapsulated Enterococcus fecalis and the extract of Camellia oleifera seed on growth performance, intestinal morphology, and intestinal mucosal immune functions in broiler chickens", ANIMAL FEED SCIENCE AND TECHNOLOGY, vol. 212, février 2016 (2016-02), pages 42-51, XP002786335,
- Weerasigha A.S. et al.: Effects of Fenugreek (Trigonella foenum-graecum L.) Seed Powder onGrowth Performance, Visceral Organ Weight, Serum Cholesterol Levelsand the Nitrogen Retention of Broiler Chicken Tropical agricultural research, vol. 24, no. 3 2013, pages 289-295, XP002786336, Extrait de l'Internet: URL:https://pdfs.semanticscholar.org/209e/ f0d9ac22fd03ec5831720c443e23dff8978d.pdf [extrait le 2018-11-06]
- DATABASE BIOSIS [Online] BIOSCIENCES INFORMATION SERVICE, PHILADELPHIA, PA, US; avril 2003 (2003-04), SCHMEDA-HIRSCHMANN G ET AL: "Free-radical scavengers and antioxidants from Peumus boldus Mol. ("Boldo").", XP002786337, Database accession no. PREV200300181007

## Description

L'invention concerne un additif alimentaire destiné à l'animal, en particulier aux volailles. Elle se rapporte également à l'utilisation de cet additif alimentaire pour la prévention ou le traitement de la coccidiose de l'animal, en particulier de la volaille.

La coccidiose est une pathologie parasitaire pouvant affecter plusieurs espèces animales comme les volailles (par exemple poulets, poules, dindes, canards, pintades, oies), les ruminants (par exemple bovins, ovins, caprins), les porcs et les lapins.

Les parasites responsables de cette pathologie sont les coccidies qui sont des protozoaires du genre *Eimeria.* Il en existe de nombreuses espèces plus ou moins pathogènes. Les espèces les plus pathogènes présentes chez le poulet sont *Eimeria acervulina*, *Eimeria maxima* et *Eimeria tenella.*

Le cycle de développement du parasite est divisé en plusieurs phases, se déroulant en partie dans le milieu extérieur, mais aussi dans l'intestin des animaux infestés causant ainsi des lésions à différents endroits du tube digestif.

Chez le poulet :
- *Eimeria acervulina* va causer des lésions au niveau du duodénum (partie supérieure de l'intestin grêle) ;
- *Eimeria maxima* causera la présence de lésions au niveau du jéjunum et de l'iléon, et enfin
- *Eimeria tenella* induira la présence de lésions dans les caeca.

L'infestation se traduit alors par une diminution de la consommation d'eau et de nourriture, une perte de poids, mais aussi par la présence de diarrhées. Les volailles sont visiblement malades. Les infestations les plus graves peuvent conduire à la mort de l'animal.

Il existe de nombreux coccidiostatiques qui ont été proposés afin de traiter la coccidiose.

Tout d'abord, des antibiotiques permettent de lutter contre cette pathologie. En effet, il en existe une diversité permettant de lutter contre la coccidiose, préparés par exemple à partir de Streptomyces comme cela est décrit dans la demande de brevet FR 2 356 667 A1 ou encore à base d'éther acide polycyclique obtenu par fermentation du microorganisme Actinomadura comme cela est divulgué dans la demande internationale WO93/12800 A1.

Par ailleurs, des molécules synthétiques permettent également de traiter la coccidiose. Il peut s'agir de :
- pyroles comme cela est décrit dans la demande internationale WO01/34 632 A2,
- purines comme cela est décrit dans la demande EP 76 127 A2, ou encore de
- dérivés de la riboflavine comme cela est divulgué dans la demande de brevet FR 2 368 956 A1.

Aussi, il existe des vaccins contre les protozoaires. Par exemple, la demande de brevet EP 47 662 A2 décrit un vaccin obtenu à partir d'une souche atténuée d'*Eimeria necatrix* qui permet de traiter les volailles contre la coccidiose. Le vaccin peut être distribué dans la nourriture ou l'eau de boisson de l'animal.

Enfin, des formulations à base d'actifs végétaux permettent d'agir contre la coccidiose en élevage. A cet égard, on peut citer :
- des formulations élaborées à base d'artémisinine telles que décrites par exemple dans la demande internationale WO 00/04025 A1 ;
- des formulations à base de coque de noix de cajou telles que décrites par exemple dans la demande de brevet EP 1103 190 A1 ;
- des formulations à bases de tannins hydrolysables telles que décrites par exemple dans la demande internationale WO 2014/004761 A2 ;
- des formulations à base de sapogénines telles que décrites dans la demande de brevet EP 1 082 909 A1 ;
- le mélange comprenant un extrait de graine de fenugrec, un extrait d'eucalyptus et un extrait de feuilles de boldo tel que décrit dans la demande internationale WO 03/055328 A1.

Du fait des effets dévastateurs des infections de coccidiose rappelés ci-dessus, on est constamment à la recherche de nouveaux coccidiostatiques plus performants.

Les saponines sont des métabolites secondaires naturellement produits par des plantes. Les saponines sont très fréquemment présentes dans les racines, tiges, feuilles, graines ou fruits de plantes. Il s'agit d'hétérosides complexes composés d'un sucre associé à un stéroïde, un stéroïde alcaloïde (c'est-à-dire comportant une fonction azotée) ou un triterpène. Du fait de la diversité de structure des glucides et des aglycones, il existe de nombreuses saponines.

Les inventeurs de la présente invention ont découvert de manière tout à fait surprenante que l'administration d'une formulation comprenant l'association des saponines de deux plantes que sont *Camellia oleifera* et *Trigonella foenum-graecum* avec un composé ayant des propriétés antioxydantes dans des quantités déterminées avait un effet coccidiostatique sur les animaux, en particulier sur les volailles. En effet, une réduction des lésions intestinales et de l'excrétion d'oocystes a été observée lors de tests réalisés chez la volaille. Les inventeurs ont ainsi constaté un effet synergique pour le traitement ou la prévention de la coccidiose de la volaille entre les saponines des deux plantes précitées et un composé ayant des propriétés antioxydantes lorsqu'ils étaient administrés dans des quantités déterminées.

La présente invention est décrite ci-dessous en prenant appui sur l'exemple de la volaille sans que cela ne limite sa portée.

La présente invention a ainsi pour premier objet un additif alimentaire destiné à l'animal, en particulier à la volaille, qui se caractérise en ce qu'il comprend, en pourcentages massiques exprimés par rapport à la masse totale dudit additif, au moins :
- entre 0,02% et 10%, de préférence entre 0,1% et 4,5%, de saponines de la plante *Camellia oleifera* ;
- entre 0,2% et 19%, de préférence entre 0,6% et 9,5%, de saponines de la plante *Trigonella foenum-graecum* ; et
- entre 1% et 25%, de préférence entre 1% et 15%, d'un composé ayant des propriétés antioxydantes.

L'additif alimentaire selon l'invention présente en outre l'avantage d'agir sur les performances zootechniques de l'animal puisqu'il va contribuer, de par la diminution de l'infestation due aux coccidies et la lutte contre le stress oxydatif, à maintenir ces performances.

Le *Camellia oleifera* est un arbre originaire de Chine, appartenant à la famille des *Theaceae.* Le *Camellia oleifera* contient des saponines dans ses graines, ses feuilles, ses tiges et ses racines.

Le *Trigonella foenum-graecum,* aussi connu sous la dénomination de fenugrec, est une plante herbacée de la famille des *Fabaceae,* et plus particulièrement des protéagineux. Le *Trigonella foenum-graecum* contient des saponines dans ses graines, ses feuilles, ses tiges et ses racines.

C'est pourquoi, l'additif alimentaire selon l'invention peut comprendre au moins :
- des graines, des feuilles, des tiges ou des racines, prises seules ou en mélanges de celles-ci, de *Camellia oleifera* qui contiennent des saponines ;
- des graines, des feuilles, des tiges ou des racines, prises seules ou en mélanges de celles-ci, de *Trigonella foenum-graecum* qui contiennent des saponines ;
- le composé ayant des propriétés antioxydantes.

Les graines, les feuilles, les tiges et les racines de *Camellia oleifera* et de *Trigonella foenum-graecum* peuvent être sous forme de poudre.

Dans un mode de réalisation de l'invention, l'additif alimentaire comprend un extrait de graines, feuilles, tiges ou racines, prises seules ou en mélanges de celles-ci, de *Camellia oleifera* qui contient lesdites saponines. L'extrait de graines, feuilles, tiges ou racines, prises seules ou en mélanges de celles-ci, de *Camellia oleifera* peut être sous forme de poudre.

Dans un mode de réalisation de l'invention, l'additif alimentaire comprend un extrait de graines, feuilles, tiges ou racines, prises seules ou en mélanges de celles-ci, de *Trigonella foenum-graecum.* L'extrait de *Trigonella foenum graecum* peut être sous forme de poudre.

Ainsi, dans un mode de réalisation de l'invention, l'additif alimentaire comprend :
- un extrait de graines, feuilles, tiges ou racines, prises seules ou en mélanges de celles-ci, de *Camellia oleifera* qui contient des saponines ;
- un extrait de graines, feuilles, tiges, ou racines, prises seules ou en mélanges de celles-ci, de *Trigonella foenum-graecum* qui contient des saponines ;
- le composé ayant des propriétés antioxydantes.

L'obtention d'un extrait d'une plante (par exemple une plante de *Camellia oleifera* ou de *Trigonella foenum-graecum*) est parfaitement à la portée de l'homme du métier qui maîtrise les techniques d'extraction sur les plantes, afin d'en extraire des substances actives qui incluent notamment les saponines.

Les techniques d'extraction de substances actives de plantes comprennent généralement les étapes suivantes :
- un traitement de broyage,
- une extraction par un solvant, par exemple un solvant polaire tel que l'eau ou un alcool ;
- un ou plusieurs lavages, par exemple avec un solvant non polaire.

L'extraction de plantes permet de dissoudre les substances actives qu'elles contiennent dans le solvant. Après évaporation du solvant, on obtient un extrait de la plante qui contient de manière concentrée les substances actives, notamment les saponines.

Dans un mode de réalisation avantageux de l'invention, les extraits de plantes sont obtenus selon un procédé qui comprend au moins les étapes suivantes :
- une étape de macération des matières premières de la plante dans une solution aqueuse contenant entre 5% et 80% en masse d'éthanol par rapport à la masse totale de la solution ;
- une étape de concentration au cours de laquelle les matières premières sont standardisées en matière sèche par séchage ;
- une étape de broyage de manière à obtenir un extrait de la plante sous forme de poudre.

Cette préparation des extraits des plantes présente l'avantage d'améliorer la biodisponibilité des substances actives qu'elles contiennent, notamment des saponines.

Dans un mode de réalisation de l'invention, l'additif peut en outre comprendre des saponines qui peuvent être choisies parmi les saponines des plantes suivantes : *Aesculus hippocastanum, Argania spinosa, Calendula officinalis, Camellia sinensis, Chenopodium quinoa, Gypsophila paniculata, Medicago sativa, Melilotus officinalis, Primula veris, Quillaja saponaria, Saponaria officinalis, Saponaria vaccaria, et Yucca schidigera,* prises seules ou en mélange de celles-ci.

L'additif alimentaire selon l'invention peut ainsi comprendre un extrait d'au moins une de ces plantes de telle sorte qu'il contienne de manière concentrée les saponines de ces plantes.

Le composé ayant des propriétés antioxydantes a avantageusement une activité antioxydante supérieure ou égale à 2 500 µmol trolox/g, ladite activité antioxydante étant mesurée par une méthode décrite dans la publication intitulée « DPPH antioxidant assay revisited » de Om P. Sharma et Tej K. Bhat, Food Chemistry, Volume 113, n°4, 15 Avril 2009, pages 1202-1205. « DDPH » est l'abréviation du composé chimique : 2,2-diphényl-1-picrylhydrazyle.

De préférence, le composé ayant des propriétés antioxydantes a une activité antioxydante comprise entre 2 500 et 25 000 µmol trolox/g, plus préférentiellement entre 3 000 et 10 000 µmol trolox/g.

Avantageusement, le composé ayant des propriétés antioxydantes est contenu dans un extrait de plante.

Dans ce mode de réalisation de l'invention, l'additif alimentaire peut comprendre entre 1% et 50%, de préférence entre 1% et 25%, d'un extrait d'au moins une plante qui contient ledit composé ayant des propriétés antioxydantes.

L'extrait de plante qui contient ledit composé ayant des propriétés antioxydantes peut être choisi parmi les extraits des plantes de *Castanea sativa, Camellia sinensis, Rosmarinus officinalis, Vitis vinifera,* ou encore *Olea europea,* pris seuls ou en mélange de ceux-ci.

Ainsi, dans un mode de réalisation de l'invention, l'additif alimentaire comprend un extrait d'au moins une plante choisie parmi *Castanea sativa, Camellia sinensis, Rosmarinus officinalis, Vitis vinifera* ou d'*Olea europea,* prises seules ou en mélanges de celles-ci, et qui contient ledit composé ayant des propriétés antioxydantes.

De manière préférée, il s'agit d'un extrait de plante de *Castanea sativa.*

Le composé ayant des propriétés antioxydantes permet de neutraliser les radicaux libres produits lors d'un stress oxydatif, notamment d'un stress oxydatif ayant pour origine la coccidiose. En effet, la coccidiose induit la production de radicaux libres.

Dans un mode de réalisation de l'additif alimentaire, le ratio du pourcentage massique des saponines de *Camellia oleifera* sur le pourcentage massique des saponines de *Trigonella foenum-graecum* est compris entre 0,02 : 19 et 10 : 0,2, de préférence entre 0,1 : 9,5 et 4,5 : 0,6.

Dans un mode de réalisation de l'additif alimentaire, le ratio de la somme des pourcentages massiques des saponines de *Camellia oleifera* et de *Trigonella foenum-graecum* sur le pourcentage massique du composé ayant des propriétés antioxydantes est compris entre 0,22 : 25 et 29 : 1, de préférence entre 0,7 : 15 et 14 : 1.

Lorsque l'additif alimentaire comprend un extrait de graines, feuilles, tiges, ou racines, prises seules ou en mélanges de celles-ci, de *Camellia oleifera*, l'extrait comprend entre 1% et 50%, de préférence entre 1% et 20%, plus préférentiellement entre 5% et 15%, de saponines par rapport à la masse totale de matière sèche dudit extrait.

Lorsque l'additif alimentaire comprend un extrait de graines, feuilles, tiges, ou racines, prises seules ou en mélanges de celles-ci, de *Trigonella foenum-graecum,* l'extrait comprend entre 1% et 20%, de préférence entre 1% et 10%, de saponines par rapport à la masse totale de matière sèche dudit extrait.

Pour compléter l'additif alimentaire en masse à 100%, il peut en outre contenir au moins un composé choisi parmi des excipients tels que la silice, l'argile, des conservateurs, des farines, ainsi que des co-produits céréaliers (par exemple du blé broyé ou du son).

Le pourcentage massique de ce composé peut être compris entre 0,01% et 75%, de préférence entre 0,01% et 50%, par rapport à la masse totale dudit additif.

De manière préférée, l'additif alimentaire comprend en pourcentages massiques exprimés par rapport à la masse totale dudit additif :
- entre 2 % et 50 %, de préférence entre 2% et 30%, d'un extrait de graines, feuilles, tiges ou racines, prises seules ou en mélanges de celles-ci, de *Camellia oleifera ;*
- entre 20 % et 95 %, de préférence entre 60% et 95%, d'un extrait de graines, feuilles, tiges ou racines, prises seules ou en mélanges de celles-ci, de *Trigonella foenum-graecum ;*
- entre 1% et 25%, de préférence entre 1% et 15%, du composé ayant des propriétés antioxydantes ou entre 1% et 50%, de préférence entre 1 et 25%, d'un extrait de plante qui contient ledit composé ayant des propriétés antioxydantes ;
- optionnellement entre 0,01% et 75%, de préférence entre 0,01% et 50%, d'un composé choisi parmi les excipients tels que la silice, l'argile, les conservateurs, les farines et les co-produits céréaliers.

L'additif alimentaire selon l'invention peut se présenter sous forme de pellet, de poudre ou de mélange poudreux.

L'additif alimentaire de la présente invention peut être intégré à l'aliment de volailles à raison de 10 ppm à 1000 ppm, de préférence à raison de 250 ppm.

Dans un mode de réalisation de l'invention, l'additif alimentaire est intégré dans l'aliment de l'animal à raison de 0,01 à 1,00 kg, de préférence de 0,10 à 0,50 kg, par tonne d'aliment de l'animal.

Dans un mode de réalisation de l'invention, l'additif alimentaire est intégré dans l'aliment à raison de 0,09 à 9,00 g, de préférence de 0,9 à 4,5 g/100 kg de poids vif de volaille par jour (pour des animaux de 1,5 kg de poids vif consommant 0,135 kg d'aliment complet par jour).

Lorsque l'additif alimentaire comprend uniquement des composés à base de plantes riches en métabolites secondaires, il est alors totalement d'origine naturelle et peut être utilisé jusqu'à l'abattage de l'animal, sans période de retrait préalable nécessaire.

L'invention a ainsi pour objet un aliment pour animal, de préférence une volaille, qui se caractérise en ce qu'il comprend entre 0,01 et 1,00 kg, de préférence entre 0,10 et 0,50 kg, d'additif alimentaire tel que décrit ci-dessus par tonne d'aliment.

L'aliment pour animal peut comprendre :
- entre 2,5 et 100 g/tonne d'aliment, de préférence entre 2,5 et 25 g/tonne d'aliment, de saponines de *Camellia oleifera* ou entre 1 et 2000 g/tonne d'aliment, de préférence entre 3 et 650 g/tonne d'aliment, d'un extrait de *Camellia oleifera* ;
- entre 2,5 et 100 g/tonne d'aliment, de préférence entre 2,5 et 25 g/tonne d'aliment, de saponines de *Trigonella foenum-graecum* ou entre 25 et 10 000 g/tonne d'aliment, de préférence entre 25 et 500 g/tonne d'aliment, d'un extrait de *Trigonella foenum-graecum;*
- entre 1 et 25 g/tonne d'aliment, de préférence entre 1 et 15 g/tonne d'aliment, du composé ayant des propriétés antioxydantes ou entre 1 et 100 g/tonne d'aliment, de préférence entre 1 et 50 g/tonne d'aliment, d'un extrait d'une plante qui contient ledit composé ayant des propriétés antioxydantes.

De par sa propriété anticoccidienne, l'additif alimentaire de l'invention peut être incorporé dans la composition d'un aliment d'un animal (de préférence une volaille) qui est destiné à avoir une action coccidiostatique. L'additif alimentaire va pouvoir agir sur certains facteurs de risques de la maladie mis en évidence comme le statut immunitaire de l'animal et le stress oxydatif grâce au composé aux propriétés antioxydantes. Un autre facteur de risque identifié est la présence d'aliments dits « favorisant » la coccidiose dans la ration alimentaire de l'animal. Il peut s'agir de régimes riches en protéines et/ou qui peuvent comprendre des acides gras essentiels, des vitamine B et D ou encore du calcium (si sa teneur massique dans la ration est supérieure à 1%). L'apport de l'additif alimentaire sert à contrecarrer la présence de ces composants à risque.

L'additif alimentaire peut également être utilisé dans le but qu'il agisse directement sur le développement des coccidies, ou encore sur l'altération de la muqueuse intestinale, sur l'excrétion d'oocystes et donc sur la recontamination de l'environnement, et enfin sur la diminution des performances zootechniques.

L'invention a ainsi pour objet l'additif alimentaire ou l'aliment tels que décrits ci-dessus pour le traitement préventif ou curatif de la coccidiose, de préférence la coccidiose de la volaille.

L'invention sera mieux comprise à l'aide de la description détaillée d'expérimentations qui sont exposées ci-dessous en référence au dessin annexé représentant des résultats de données expérimentales relatives à l'administration à des animaux d'aliments selon l'invention et d'aliments comparatifs.

### DESCRIPTION DES FIGURES

La figure 1 représente un graphique sur lequel sont détaillés les pourcentages relatifs d'amélioration de l'indice de consommation qui ont été déterminés avec différents essais réalisés sur des poulets à qui on a administré différents aliments à base d'extraits de *Trigonella foenum-graecum* et de *Camellia oleifera.*

La figure 2 représente un graphique représentant les scores lésionnels causés par 3 espèces du parasite Eimeria sur 5 lots de poulets à qui on a administré différents aliments.

La figure 3 représente un graphique représentant les scores lésionnels causés par 3 espèces du parasite Eimeria sur 5 lots de poulets à qui on a administré différents aliments.

### PARTIE EXPERIMENTALE

### I - Effet synergique de l'association de Camellia oleifera et de Trigonella foenum-graecum sur l'indice de consommation de poulets infectés de coccidiose

Les résultats présentés ci-après ont été obtenus par synthèse des résultats de 4 essais expérimentaux détaillés ci-dessous.

### Essai N°1 :

Les animaux expérimentaux utilisés pour réaliser l'essai n°1 étaient des poulets de chair. 224 poulets de race Cobb 500 ont fait l'objet de l'étude.

Les 2 lots témoins suivants ont été établis :
- 1^{er} lot témoin T1 : un groupe de 56 poulets qui n'étaient pas infectés par la coccidiose.
- 2^{ème} lot témoin T2 : un groupe de 56 poulets qui étaient infectés par les coccidies *Eimeria acervulina*, *Eimeria maxima* et *Eimeria tenella.*

Par ailleurs, les lots A et B suivants ont été établis :
- Lot A : un groupe de 56 poulets ayant tous été infectés par les coccidies *Eimeria acervulina*, *Eimeria maxima* et *Eimeria tenella,* et à qui on a administré journalièrement un extrait de *Camellia oleifera* intégré à l'aliment à hauteur de 0,25 kg/tonne pendant 20 jours ;
- Lot B : un groupe de 56 poulets ayant tous été infectés par les coccidies *Eimeria acervulina*, *Eimeria maxima* et *Eimeria tenella,* et à qui on a administré journalièrement un extrait de *Trigonella foenum-graecum* intégré à l'aliment à hauteur de 0,25 kg/tonne pendant 20 jours.

### Essai N°2 :

Les animaux expérimentaux pris en compte pour la réalisation de la synthèse des résultats sont uniquement les deux groupes témoins pour cet essai n°2. 96 poulets de race Ross 308 ont fait l'objet de l'étude.

Les 2 lots témoins suivants ont été établis :
- 1^{er} lot témoin T1 : un groupe de 48 poulets qui n'étaient pas infectés par la coccidiose.
- 2^{ème} lot témoin T2 : un groupe de 48 poulets qui étaient infectés par les coccidies *Eimeria acervulina*, *Eimeria maxima* et *Eimeria tenella.*

### Essai N°3 :

Les animaux expérimentaux utilisés pour réaliser l'essai n°3 étaient des poulets de chair. 392 poulets de race Cobb 500 ont fait l'objet de l'étude.

Les 2 lots témoins suivants ont été établis :
- 1^{er} lot témoin T1 : un groupe de 56 poulets qui n'étaient pas infectés par la coccidiose.
- 2^{ème} lot témoin T2 : un groupe de 56 poulets qui étaient infectés par les coccidies *Eimeria acervulina*, *Eimeria maxima* et *Eimeria tenella.*

Par ailleurs, les lots A à E suivants ont été établis :
- Lot A : un groupe de 56 poulets ayant tous été infectés par les coccidies *Eimeria acervulina*, *Eimeria maxima* et *Eimeria tenella,* et à qui on a administré journalièrement un extrait de *Camellia oleifera* intégré à l'aliment à hauteur de 0,25 kg/tonne pendant 20 jours ;
- Lot B : un groupe de 56 poulets ayant tous été infectés par les coccidies *Eimeria acervulina*, *Eimeria maxima* et *Eimeria tenella,* et à qui on a administré journalièrement un extrait de *Trigonella foenum-graecum* intégré à l'aliment à hauteur de 0,25 kg/tonne pendant 20 jours ;
- Lot C : un groupe de 56 poulets ayant tous été infectés par les coccidies *Eimeria acervulina*, *Eimeria maxima* et *Eimeria tenella,* et à qui on a administré journalièrement pendant 20 jours :
   ∘ un extrait de *Camellia oleifera* intégré à l'aliment à hauteur de 0,1875 kg/tonne.
   ∘ un extrait de *Trigonella foenum-graecum* intégré à l'aliment à hauteur de 0,0625 kg/tonne.
      Le ratio de la quantité de *Camellia oleifera* sur celle de *Trigonella foenum-graecum* était de 3 : 1 ;
- Lot D : un groupe de 56 poulets ayant tous été infectés par les coccidies *Eimeria acervulina*, *Eimeria maxima* et *Eimeria tenella,* et à qui on a administré journalièrement pendant 20 jours :
   ∘ un extrait de *Camellia oleifera* intégré à l'aliment à hauteur de 0,125 kg/tonne.
   ∘ un extrait de *Trigonella foenum-graecum* intégré à l'aliment à hauteur de 0,125 kg/tonne.
      Le ratio de la quantité de *Camellia oleifera* sur celle de *Trigonella foenum-graecum* était de 1:1 ;
- Lot E : un groupe de 56 poulets ayant tous été infectés par les coccidies *Eimeria acervulina*, *Eimeria maxima* et *Eimeria tenella,* et à qui on a administré journalièrement pendant 20 jours :
   ∘ un extrait de *Camellia oleifera* intégré à l'aliment à hauteur de 0,0625 kg/tonne.
   ∘ un extrait de *Trigonella foenum-graecum* intégré à l'aliment à hauteur de 0,1875 kg/tonne.
      Le ratio de la quantité de *Camellia oleifera* sur celle de *Trigonella foenum-graecum* était de 1 : 3.

### Essai N°4 :

Les animaux expérimentaux utilisés pour réaliser l'essai n°4 étaient des poulets de chair. 336 poulets de race Ross 308 ont fait l'objet de l'étude.

Les 2 lots témoins suivants ont été établis :
- 1^{er} lot témoin T1 : un groupe de 48 poulets qui n'étaient pas infectés par la coccidiose.
- 2^{ème} lot témoin T2 : un groupe de 48 poulets qui étaient infectés par les coccidies *Eimeria acervulina*, *Eimeria maxima* et *Eimeria tenella.*

Par ailleurs, les lots A à E suivants ont été établis :
- Lot A : un groupe de 48 poulets ayant tous été infectés par les coccidies *Eimeria acervulina*, *Eimeria maxima* et *Eimeria tenella,* et à qui on a administré journalièrement un extrait de *Camellia oleifera* intégré à l'aliment à hauteur de 0,25 kg/tonne pendant 20 jours ;
- Lot B : un groupe de 48 poulets ayant tous été infectés par les coccidies *Eimeria acervulina*, *Eimeria maxima* et *Eimeria tenella,* et à qui on a administré journalièrement un extrait de *Trigonella foenum-graecum* intégré à l'aliment à hauteur de 0,25 kg/tonne pendant 20 jours ;
- Lot C : un groupe de 48 poulets ayant tous été infectés par les coccidies *Eimeria acervulina*, *Eimeria maxima* et *Eimeria tenella,* et à qui on a administré journalièrement pendant 20 jours :
   ∘ un extrait de *Camellia oleifera* intégré à l'aliment à hauteur de 0,1875 kg/tonne.
   ∘ un extrait de *Trigonella foenum-graecum* intégré à l'aliment à hauteur de 0,0625 kg/tonne.
      Le ratio de la quantité de *Camellia oleifera* sur celle de *Trigonella foenum-graecum* était de 3 : 1 ;
- Lot D : un groupe de 48 poulets ayant tous été infectés par les coccidies *Eimeria acervulina*, *Eimeria maxima* et *Eimeria tenella,* et à qui on a administré journalièrement pendant 20 jours :
   ∘ un extrait de *Camellia oleifera* intégré à l'aliment à hauteur de 0,125 kg/tonne.
   ∘ un extrait de *Trigonella foenum-graecum* intégré à l'aliment à hauteur de 0,125 kg/tonne.
      Le ratio de la quantité de *Camellia oleifera* sur celle de *Trigonella foenum graecum* était de 1:1 ;
- Lot E : un groupe de 48 poulets ayant tous été infectés par les coccidies *Eimeria acervulina*, *Eimeria maxima* et *Eimeria tenella,* et à qui on a administré journalièrement pendant 20 jours :
   ∘ un extrait de *Camellia oleifera* intégré à l'aliment à hauteur de 0,0625 kg/tonne.
   ∘ un extrait de *Trigonella foenum-graecum* intégré à l'aliment à hauteur de 0,1875 kg/tonne.
      Le ratio de la quantité de *Camellia oleifera* sur celle de *Trigonella foenum-graecum* était de 1 : 3.

L'inoculation par les coccidies a eu lieu au 14^{ème} jour pour les essais n°1 et 3 et au 16^{ème} jour pour les essais n°2 et 4.

L'indice de consommation a été mesuré pendant la période du 14^{ième} au 20^{ième} jour d'essai. Il correspond au rapport entre la quantité d'aliment consommée et le gain de poids de l'animal.

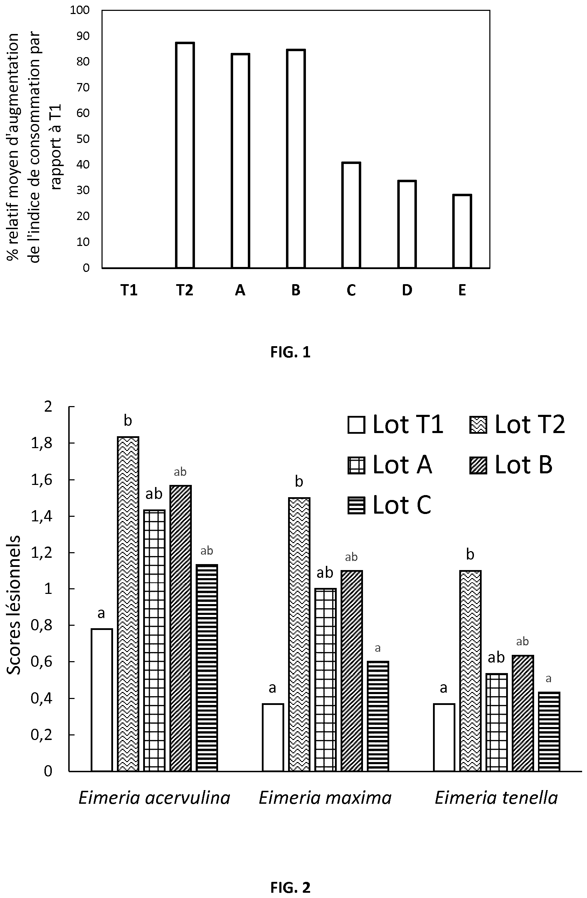

Pour chacun des lots détaillés ci-dessus, et ce pour les 4 essais, le pourcentage relatif d'augmentation de l'indice de consommation par rapport au témoin T1 (non infecté) sur la période du 14^{ème} au 20^{ème} jour d'essai a été calculé. La moyenne de ces pourcentages relatifs a été calculée pour les groupes ayant reçu le même traitement, tous essais confondus. On a alors obtenu un pourcentage relatif moyen d'augmentation de l'indice de consommation par rapport au témoin T1 pour chacun des traitements testés. Ceux-ci sont présentés sur le graphique constituant la figure 1.

On observe que l'infection des animaux du groupe Témoin T2 cause une augmentation de l'indice de consommation de 87,32% par rapport au Témoin T1.

Cette augmentation est atténuée par les différentes administrations de *Camellia oleifera et Trigonella foenum-graecum* qui ont été testées sur les lots de poulets A à E.

Il faut alors noter que l'administration seule de *Camellia oleifera* ou de *Trigonella foenum-graecum* réduit très peu l'indice de consommation qui a été augmenté à cause de l'infection des volailles par le parasite *Eimeria.*

Avec une administration seule de *Camellia oleifera*, on observe une augmentation de 83,07% de l'indice de consommation. Cela signifie que cette administration permet seulement de réduire de 4,25% l'indice de consommation par rapport au témoin T2.

De même, avec une administration seule de *Trigonella foenum-graecum,* on observe une augmentation de 84,62% de l'indice de consommation par rapport au Témoin T1. Cela signifie que cette administration a permis de réduire l'indice de consommation de 2,70% par rapport au Témoin T2.

Cependant, si l'on s'intéresse aux effets de l'association des deux plantes pour les lots C à E, on observe un effet synergique de cette association qui est bénéfique, car il limite de manière plus importante l'augmentation de l'indice de consommation chez les animaux infectés, et ce quel que soit le ratio.

En effet, l'indice de consommation pour les poulets des lots C à E à qui on a administré les plantes *Camellia oleifera* et *Trigonella foenum-graecum* a augmenté de respectivement 40,83%, 33,66% et 28,33% par rapport au témoin T1. Cela est bien plus faible que pour les poulets des lots A et B. Les réductions de l'indice de consommation induites par l'administration combinée de *Camellia oleifera* et *Trigonella foenum-graecum* sont respectivement de 46,49%, 53,66% et 58,99% par rapport au témoin T2.

Cette expérimentation a mis en évidence l'effet de synergie entre *Camellia oleifera* et *Trigonella foenum-graecum,* c'est-à-dire l'effet de l'association de ces deux plantes est plus important que la somme des effets individuels de chacune de ces plantes.

### II - Amélioration de la capacité hémolytique par l'association de Camellia oleifera et Trigonella foenum-graecum

Un second test réalisé in vitro portant sur la capacité hémolytique des plantes *Camellia oleifera* et *Trigonella foenum-graecum* a permis de confirmer la présence d'un effet synergique entre ces deux plantes.

Le test de capacité hémolytique consiste à mettre des échantillons de *Camellia oleifera* et *Trigonella foenum-graecum* au contact d'une solution d'hématies afin d'observer la capacité de lyse des hématies de l'échantillon testé.

On déduit de cet essai une dose hémolytique « DH50 » qui est la dose permettant de lyser 50% des hématies de solution.

Ce test permet de mesurer l'effet d'un actif sur la membrane cellulaire de globules rouges qui ont une structure similaire à la membrane cellulaire des coccidies. En effet, la membrane des globules rouges tout comme celle des coccidies comporte des stérols sur lesquels se fixe l'actif testé (par exemples les saponines testées) ; ce qui entraîne une déstabilisation membranaire, puis une lyse cellulaire. C'est pourquoi, la capacité hémolytique d'un actif testé (par exemple les saponines testées) est corrélée à sa capacité coccidiolytique.

La DH50 des plantes seules a tout d'abord été déterminée, puis les DH50 d'associations des deux plantes selon les ratios (tels que détaillés dans le tableau 1 ci-dessous) de la masse de *Camellia oleifera* sur celle de *Trigonella foenum-graecum* ont été calculées théoriquement avant d'être déterminées également.

Pour chacun des ratios, la DH50 théorique a été calculée à partir des DH50 déterminées pour chaque plante seule, en fonction de leurs régressions linéaires et de leurs rendements d'extractions respectifs.

Le tableau 1 détaille les données de DH50 calculée et observée en fonction du ratio considéré.

**Tableau 1 détaillant les données de DH50 théorique et observée en fonction du ratio considéré**

| | **DH50 (µg/mL) théorique** | **DH50 (µg/mL) observée** | **Différence entre DH50 théorique et DH50 observée (%)** |
|---|---|---|---|
| **Ratio = 1** | 2300 | 734 | -68,09% |
| **Ratio = 2** | 1560 | 483 | -69,04% |
| **Ratio = 3** | 935 | 371 | -60,32% |

On observe que pour chacun des ratios testés, la DH50 théorique est bien plus élevée que la DH50 observée.

En effet, pour le ratio 1, on observe que la DH50 mesurée lors du test est plus faible de 68,09% par rapport à la DH50 théorique. Pour le ratio 2, elle est plus faible de 69,04% et pour le ratio 3, elle est plus faible de 60,32%.

Le fait que les DH50 observées soient plus faibles que les DH50 théoriques signifie que l'association des deux plantes *Camellia oleifera* et *Trigonella foenum-graecum* présente une efficacité supérieure à ce que l'on pouvait attendre selon les calculs théoriques.

Cette expérimenation confirme également que l'association de ces deux plantes a un effet synergique.

### III - Effet synergique de l'association de Camellia oleifera, Trigonella foenum-graecum et Castanea sativa sur les scores lésionnels de poulets infectés par la coccidiose

Comme expliqué ci-dessus, la gravité de la coccidiose est mesurée par l'importance des lésions au niveau intestinal. En effet, les lésions de la paroi du tube digestif sont consécutives au cycle de développement intracellulaire du parasite.

L'oocyste est une forme de résistance du parasite lorsqu'il est dans l'atmosphère libre. Suite à l'ingestion d'oocystes, les poulets vont contracter la maladie car le parasite va se multiplier au sein de son hôte, puis le poulet va rejeter dans les fientes des oocystes qui vont contaminer d'autres poulets.

### Essai N°5 :

Les animaux expérimentaux utilisés pour réaliser l'essai N°5 étaient des poulets de chair. 500 poulets de race Cobb 500 ont fait l'objet de l'étude.

Les 2 lots témoins suivants ont été établis :
- 1^{er} lot témoin T1 : un groupe de 100 poulets qui n'étaient pas infectés par la coccidiose.
- 2^{ème} lot témoin T2 : un groupe de 100 poulets qui étaient infectés par les coccidies *Eimeria acervulina*, *Eimeria maxima, Eimeria tenella.*

Par ailleurs, les lots A, B et C suivants ont été établis :
- Lot A : un groupe de 100 poulets ayant tous été infectés par les coccidies *Eimeria acervulina*, *Eimeria maxima, Eimeria tenella,* et à qui on a administré journalièrement pendant 20 jours :
   - un extrait de *Trigonella foenum-graecum* intégré à l'aliment à hauteur de 0,240 kg/tonne ;
   - un extrait de *Castanea sativa* intégré à l'aliment à hauteur de 0,010 kg/tonne.
- Lot B : un groupe de 100 poulets ayant tous été infectés par les coccidies Eimeria *acervulina*, *Eimeria maxima, Eimeria tenella,* et à qui on a administré journalièrement pendant 20 jours un mélange d'extraits de *Trigonella foenum-graecum* et de *Yucca schidigera* et intégré à l'aliment à hauteur de 0,250 kg/tonne soient 11,25 g de saponines /tonne d'aliment. Dans ce mélange : 45 % des saponines totales étaient apportées par l'extrait de *Trigonella foenum-graecum* et 55 % des saponines totales étaient apportées par l'extrait de *Yucca schidigera.*
- Lot C : un groupe de 100 poulets ayant tous été infectés par les coccidies *Eimeria acervulina*, *Eimeria maxima, Eimeria tenella,* et à qui on a administré journalièrement pendant 20 jours :
   o un extrait de *Trigonella foenum-graecum* intégré à l'aliment à hauteur de 0,190 kg/tonne ;
   ∘ un extrait de *Camellia oleifera* intégré à l'aliment à hauteur de 0,050 kg/tonne ;
   ∘ Un extrait de *Castena sativa* intégré à l'aliment à hauteur de 0,010 kg/tonne.

Ainsi, on a administré aux lots A et B un aliment comparatif et au lot C un aliment selon l'invention.

En ce qui concerne l'infection du 2^{ème} lot témoin T2 et des lots A à C, l'inoculation par les coccidies a eu lieu au 14^{ème} jour par gavage oral des poulets avec un vaccin vivant (Coccivac®-B52 commercialisé par la société Merck) à trois fois la dose normale.

Pour chacun des 5 lots, les 100 poulets étaient répartis par dizaine dans 10 cages.

Au 21^{ème} jour, un comptage des lésions a été réalisé.

Pour ce faire, 3 poulets ont été sélectionnés dans chaque cage de manière aléatoire pour être euthanasiés afin de noter les lésions typiques pour la coccidiose selon la méthode détaillée dans la publication intitulée « Anticoccidial drugs: lesion scoring techniques in battery and floor-pen experiments with chickens » de J. Johnson et WM Reid, Exp Parasitol. août 1970; 28(1), pages 30-36, avec un score allant de 0 (pas de lésions) à 4 (lésions sévères).

La notation des lésions est une évaluation de la pathologie macroscopique et des dommages physiques causés par le parasite.

Les résultats ont été analysés par l'analyse de la variance ANOVA. Les différences ont été considérées comme significatives lorsque p était inférieur à 0,05 (avec un intervalle de confiance à 95%).

Le tableau 2 ci-dessous détaille pour les 5 lots les scores lésionnels au 21^{ème} jour qui ont été causés par les 3 espèces du parasite Emeiria suivantes : *Eimeria acervulina*, *Eimeria tenella* et *Eimeria maxima.*

**Tableau 2 détaillant les lésionnels au 21^{ième} jour pour les 5 lots de l'essai n°5**

| | Lot T1 | Lot T2 | Lot A | Lot B | Lot C |
|---|---|---|---|---|---|
| *Eimeria acervulina* | 0,778^{a} | 1,833^{b} | 1,433^{ab} | 1,567^{ab} | 1,133^{ab} |
| *Eimeria maxima* | 0,370^{a} | 1,500^{b} | 1,000^{ab} | 1,100^{ab} | 0,600^{e} |
| *Eimeria tenella* | 0,370^{a} | 1,100^{b} | 0,533^{ab} | 0,633^{ab} | 0,433^{a} |

La figure 2 est un graphique représentant les scores lésionnels causés par les 3 espèces de parasite Eimeria précitées sur les Lots T1, T2 et A à C.

La provocation volontaire de la coccidiose au sein des animaux du lot T2 a bien fonctionné puisqu'on a constaté pour ces animaux des scores lésionnels significativement supérieurs à ceux des animaux sains du lot témoin T1. Cela signifie qu'ils ont bien été rendus malades.

Effet sur lésions *Eimeria acervulina* :
Quel que soit le mélange d'extraits de plantes administré, on observe une amélioration numérique des scores lésionnels pour les lots A à C. Cependant, l'effet est plus prononcé pour le lot C à qui on a administré l'aliment selon l'invention, à savoir l'aliment comprenant un extrait de *Trigonella foenum-graecum,* un extrait de *Camellia oleifera* et un extrait de *Castena sativa.*

Effet sur les lésions *Eimeria maxima* et *tenella* :
Lorsque les animaux ont reçu le mélange composé d'un composé de *Trigonella foenum-graecum* et d'un extrait de *Castanea sativa* (à savoir les animaux du Lot A) ou le mélange composé d'un extrait de *Trigonella foenum-graecum* et d'un extrait de *Yucca schidigera* (à savoir les animaux du Lot B), aucune amélioration significative n'a été observée. En d'autres termes, on n'a pas constaté d'amélioration sur les scores lésionnels pour les animaux des Lots A et B à qui on a administré des aliments comparatifs.

Par contre, on relève que pour les animaux du Lot C à qui on a administré un aliment selon l'invention, les scores lésionnels ont été réduits de façon significative. On relève en effet des valeurs de scores lésionnels au même niveau que celles du lot témoint T1 (à savoir le lot des animaux qui étaient sains parce qu'on ne leur a pas innoculé de coccidies).

Ainsi, cette expérimentation met clairement en évidence l'effet de synergie entre les extraits des deux plantes que sont *Trigonella foenum-graecum, Camellia oleifera*, ainsi que d'un extrait de la plante *Castena sativa* qui contient des composés ayant des propriétés antioxydantes pour traiter la coccidiose

Cet effet de synergie n'a pas été observé pour des aliments comparatifs qui contenaient :
- soit un extrait d'une des deux plantes précitées (à savoir *Trigonella foenum-graecum*) en association avec l'extrait de la plante *Castena sativa -* cet aliment comparatif était donc dépourvu d'extrait de *Camellia oleifera ;*
- soit un extrait d'une des deux plantes précitées (à savoir *Trigonella foenum-graecum*) en association avec un extrait d'une autre plante *Yucca schidigera* - cet aliment comparatif était donc dépourvu d'extrait de *Camellia oleifera* et d'extrait de plante contenant un composé ayant des propriétés antioxydantes.

### Essai N°6 :

Les animaux utilisés pour réaliser l'essai N°6 étaient des poulets de chair. 240 poulets de race ROSS 308 ont fait l'objet de l'étude.

Les 2 lots témoins suivants ont été établis :
- 1^{er} lot témoin T1 : un groupe de 48 poulets qui n'étaient pas infectés par la coccidiose.
- 2^{ème} lot témoin T2 : un groupe de 48 poulets qui étaient infectés par les coccidies *Eimeria acervulina*, *Eimeria maxima, Eimeria tenella.*

Par ailleurs, les lots A, B et C suivants ont été établis :
- Lot A : un groupe de 48 poulets ayant tous été infectés par les coccidies *Eimeria acervulina*, *Eimeria maxima, Eimeria tenella,* et à qui on a administré journalièrement un extrait de *Camellia oleifera* intégré à l'aliment à hauteur de 0,25 kg/tonne pendant 22 jours ;
- Lot B : un groupe de 48 poulets ayant tous été infectés par les coccidies *Eimeria acervulina*, *Eimeria maxima, Eimeria tenella,* et à qui on a administré journalièrement un extrait de *Trigonella foenum-graecum* intégré à l'aliment à hauteur de 0,25 kg/tonne pendant 22 jours ;
- Lot C : un groupe de 48 poulets ayant tous été infectés par les coccidies *Eimeria acervulina*, *Eimeria maxima, Eimeria tenella,* et à qui on a administré journalièrement pendant 22 jours un mélange d'extraits de *Trigonella foenum-graecum* et de *Yucca schidigera* et intégré à l'aliment à hauteur de 0,250 kg/tonne soient 11,25 g de saponines /tonne d'aliment. Dans ce mélange : 45 % des saponines totales étaient apportées par l'extrait de *Trigonella foenum-graecum et* 55 % des saponines totales étaient apportées par l'extrait de *Yucca schidigera.*

En ce qui concerne l'infection du 2^{ème} lot témoin T2 et des lots A à C, l'inoculation par les coccidies a eu lieu au 16^{ème} jour par inoculation orale de 0,5 mL avec un inoculum contenant des oocystes d'*Eimeria maxima, tenella* et *acervulina.*

Pour chacun des 5 lots, les 48 poulets étaient répartis par 8 dans 6 cages.

Au 22^{ème} jour, un comptage des lésions a été réalisé.

Pour ce faire, 5 poulets ont été sélectionnés dans chaque cage de manière aléatoire pour être euthanasiés afin de noter les lésions typiques pour la coccidiose comme cela a été décrit ci-dessus dans l'essai n°5.

Le tableau 3 ci-dessous détaille pour les 5 lots les scores lésionnels au 22^{ème} jour qui ont été causés par les 3 espèces du parasite Emeiria suivantes : *Eimeria acervulina*, *Eimeria tenella* et *Eimeria maxima.*

**Tableau 3 détaillant les lésionnels au 22^{ième} jour pour les 5 lots de l'essai n°5**

| | Lot T1 | Lot T2 | Lot A | Lot B | Lot C |
|---|---|---|---|---|---|
| *Eimeria acervulina* | 0,13^{b} | 1,77^{a} | 1,90^{a} | 1,63^{a} | 1,30^{a} |
| *Eimeria maxima* | 0,03^{b} | 1,80^{a} | 1,87^{a} | 1,17^{a} | 1,43^{a} |
| *Eimeria tenella* | 0,03^{b} | 1,83^{a} | 1,40^{a} | 1,10^{a} | 1,10^{a} |

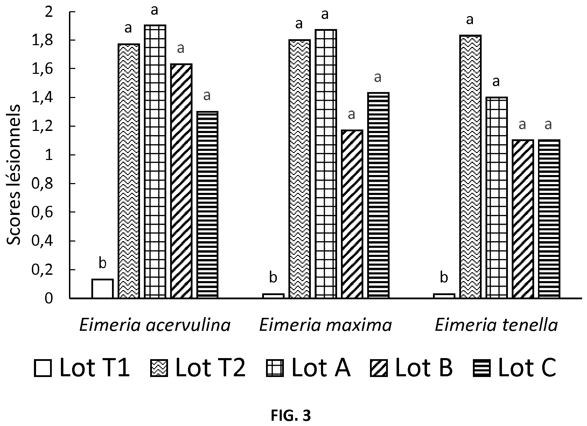

La figure 3 est un graphique représentant les scores lésionnels causés par les 3 espèces de parasite Eimeria précitées sur les Lots T1, T2 et A à C.

La provocation volontaire de la coccidiose au sein des animaux du lot T2 a bien fonctionné puisqu'on a constaté pour ces animaux des scores lésionnels significativement supérieurs à ceux des animaux sains du lot témoin T1. Cela signifie qu'ils ont bien été rendus malades.

Effet de *Camellia oleifera* (Lot A) :
L'administration de l'extrait de *Camellia oleifera* seul n'a eu aucun effet sur les scores lésionnels liés à *Eimeria acervulina* et *Eimeria maxima.* Une légère réduction numérique des lésions provoquées par *Eimeria tenella* a été constatée, mais sans que cela ne soit significatif.

Effet de la *Trigonella foenum-graecum* (Lot B) :
L'administration de l'extrait de *Trigonella foenum-graecum* seul n'a eu aucun effet sur les scores lésionnels liés à Eimeria acervulina. Une légère réduction numérique des lésions provoquées par *Eimeria tenella* et *Eimeria maxima* a été constatée, mais sans que cela ne soit significatif.

Effet de la *Trigonella foenum-graecum* et *Yucca schidigera* (Lot C) :
L'association des extraits de *Trigonella foenum-graecum* et de *Yucca schidigera* a permis une légère réduction des scores lésionnels, et ce quelle que soit la souche, mais sans que cela ne soit significatif.

Cette expérimentation réalisée avec des aliments comparatifs a montré que l'administration d'un seul extrait de *Camellia oleifera* ou de *Trigonella foenum-graecum* ou bien l'association d'un de ces extraits (à savoir l'extrait de *Trigonella foenum-graecum)* avec l'extrait d'une autre plante qu'est *Yucca schidigera* ne permet pas de traiter correctement la coccidiose.

## Revendications

1. Additif alimentaire destiné à l'animal, en particulier à la volaille, **caractérisé en ce qu'**il comprend, en pourcentages massiques exprimés par rapport à la masse totale dudit additif, au moins :
- entre 0,02% et 10%, de préférence entre 0,1% et 4,5%, de saponines de la plante *Camellia oleifera*
- entre 0,2% et 19%, de préférence entre 0,6% et 9,5%, de saponines de la plante *Trigonella foenum-graecum,* et
- entre 1% et 25%, de préférence entre 1% et 15%, d'un composé ayant des propriétés antioxydantes.

2. Additif alimentaire selon la revendication 1, **caractérisé en ce que** l'additif alimentaire comprend :
- des graines, des feuilles, des tiges ou des racines, prises seules ou en mélanges de celles-ci, de *Camellia oleifera* qui contiennent des saponines ;
- des graines, des feuilles, des tiges ou des racines, prises seules ou en mélanges de celles-ci, de *Trigonella foenum-graecum* qui contiennent des saponines ;
- le composé ayant des propriétés antioxydantes.

3. Additif alimentaire selon la revendication 1, **caractérisé en ce que** l'additif alimentaire comprend :
- un extrait de graines, feuilles, tiges ou racines, prises seules ou en mélanges de celles-ci, de *Camellia oleifera* qui contient des saponines ;
- un extrait de graines, feuilles, tiges, ou racines, prises seules ou en mélanges de celles-ci, de *Trigonella foenum-graecum* qui contient des saponines ;
- le composé ayant des propriétés antioxydantes.

4. Additif alimentaire selon l'une quelconque des revendication 1 à 3, **caractérisé en ce que** le composé ayant des propriétés antioxydantes a une activité antioxydante supérieure ou égale à 2 500 µmol trolox/g, ladite activité antioxydante étant mesurée par la méthode décrite dans la publication intitulée « DPPH antioxidant assay revisited » de Om P. Sharma et Tej K. Bhat, Food Chemistry, Volume 113, n°4, 15 Avril 2009, pages 1202-1205.

5. Additif alimentaire selon la revendication 4, **caractérisé en ce que** le composé ayant des propriétés antioxydantes a une activité antioxydante comprise entre 2 500 µmol trolox/g et 25 000 µmol trolox/g.

6. Additif alimentaire selon l'une quelconque des revendications 1 à 5, **caractérisé en ce que** l'additif alimentaire comprend entre 1% et 50%, de préférence entre 1% et 25%, d'un extrait d'au moins une plante qui contient ledit composé ayant des propriétés antioxydantes.

7. Additif alimentaire selon la revendication 6, **caractérisé en ce que** l'extrait d'une plante qui contient ledit composé ayant des propriétés antioxydantes est choisi parmi les extraits des plantes de *Castanea sativa, Camellia sinensis, Rosmarinus officinalis, Vitis vinifera* et d'*Olea europea*, pris seuls ou en mélanges de ceux-ci.

8. Additif alimentaire selon la revendication 7, **caractérisé en ce que** l'extrait de plante est un extrait de *Castanea sativa.*

9. Additif alimentaire selon l'une quelconque des revendications 1 à 8, **caractérisé en ce que** le ratio du pourcentage massique des saponines de *Camellia oleifera* sur le pourcentage massique des saponines de *Trigonella foenum-graecum* est compris entre 0,02 : 19 et 10 : 0,2, de préférence entre 0,1 : 9,5 et 4,5 : 0,6.

10. Additif alimentaire selon l'une quelconque des revendications 1 à 9, **caractérisé en ce que** le ratio de la somme des pourcentages massiques des saponines de *Camellia oleifera* et de *Trigonella foenum-graecum* sur le pourcentage massique du composé ayant des propriétés antioxydantes est compris entre 0,22 : 25 et 29 : 1, de préférence entre 0,7 : 15 et 14 : 1.

11. Additif alimentaire selon l'une quelconque des revendications 3 à 10, **caractérisé en ce qu'**il comprend en pourcentages massiques exprimés par rapport à la masse totale dudit additif :
- entre 2 % et 50 %, de préférence entre 2% et 30%, d'un extrait de graines, feuilles, tiges, ou racines, prises seules ou en mélanges de celles-ci, de *Camellia oleifera ;*
- entre 20 % et 95 %, de préférence entre 60% et 95%, d'un extrait de graines, feuilles, tiges, ou racines, prises seules ou en mélanges de celles-ci, de *Trigonella foenum-graecum ;*
- entre 1% et 25%, de préférence entre 1% et 15%, du composé ayant des propriétés antioxydantes ou entre 1% et 50%, de préférence entre 1 et 25%, d'un extrait de plante qui contient ledit composé ayant des propriétés antioxydantes ;
- optionnellement entre 0,01% et 75%, de préférence entre 0,01% et 50%, d'un composé choisi parmi les excipients tels que la silice, l'argile, les conservateurs, les farines et les co-produits céréaliers.

12. Additif alimentaire selon l'une quelconque des revendications 1 à 11, **caractérisé en ce qu'**il comprend en outre des saponines choisies parmi les saponines des plantes *Aesculus hippocastanum, Argania spinosa, Calendula officinalis, Camellia sinensis, Chenopodium quinoa, Gypsophila paniculata, Medicago sativa, Melilotus officinalis, Primula veris, Quillaja saponaria, Saponaria officinalis, Saponaria vaccaria et Yucca schidigera,* prises seules ou en mélange de celles-ci.

13. Aliment pour animal, de préférence une volaille, **caractérisé en ce qu'**il comprend entre 0,01 et 1,00 kg, de préférence entre 0,10 et 0,50 kg, d'additif alimentaire selon l'une quelconque des revendications 1 à 12 par tonne d'aliment.

14. Aliment pour animal selon la revendication 13, **caractérisé en ce qu'**il comprend :
- entre 2,5 et 100 g/tonne d'aliment, de préférence entre 2,5 et 25 g/tonne d'aliment de saponines de *Camellia oleifera* ou entre 1 et 2 000 g/tonne d'aliment, de préférence entre 3 et 650 g/tonne d'aliment, d'un extrait de *Camellia oleifera ;*
- entre 2,5 et 100 g/tonne d'aliment, de préférence entre 2,5 et 25 g/tonne d'aliment de saponines de *Trigonella foenum-graecum* ou entre 25 et 10 000 g/tonne d'aliment, de préférence entre 25 et 500 g/tonne d'aliment, d'un extrait de *Trigonella foenum-graecum ;*
- entre 1 et 25 g/tonne d'aliment, de préférence entre 1 et 15 g/tonne d'aliment, du composé ayant des propriétés antioxydantes ou entre 1 et 100 g/tonne d'aliment, de préférence entre 1 et 50 g/tonne d'aliment, d'un extrait d'une plante qui contient ledit composé ayant des propriétés antioxydantes.

15. Additif alimentaire selon l'une quelconque des revendications 1 à 12 ou aliment selon la revendication 13 ou 14 pour utilisation pour le traitement préventif ou curatif de la coccidiose, de préférence la coccidiose de la volaille.

## Patentansprüche

1. Futtermittelzusatzstoff für Tiere, insbesondere für Geflügel, **dadurch gekennzeichnet, dass** es, ausgedrückt in Gewichts-% relativ zu dem Gesamtgewicht des Zusatzstoffes, mindestens umfasst:
- zwischen 0,02% und 10%, vorzugsweise zwischen 0,1% und 4,5% Saponinen der *Camellia oleifera* Pflanze,
- zwischen 0,2% und 19%, vorzugsweise zwischen 0,6% und 9,5% Saponinen der *Trigonella foenum-graecum* Pflanze, und
- zwischen 1% und 25%, vorzugsweise zwischen 1% und 15% einer Verbindung mit antioxidativen Eigenschaften.

2. Futtermittelzusatzstoff nach Anspruch 1, **dadurch gekennzeichnet, dass** der Futtermittelzusatzstoff umfasst:
- Samen, Blätter, Stängel oder Wurzeln, einzeln oder in deren Gemischen, von *Camellia oleifera*, die Saponine enthalten;
- Samen, Blätter, Stängel oder Wurzeln, einzeln oder in deren Gemischen, von *Trigonella foenum-graecum,* welche Saponine enthalten;
- die Verbindung mit antioxidativen Eigenschaften.

3. Futtermittelzusatzstoff nach Anspruch 1, **dadurch gekennzeichnet, dass** der Futtermittelzusatzstoff umfasst:
- einen Extrakt aus Samen, Blättern, Stängeln oder Wurzeln, einzeln oder in deren Gemischen, von *Camellia oleifera,* welcher Saponine enthält;
- an Extrakt aus Samen, Blättern, Stängeln oder Wurzeln, einzeln oder in deren Gemischen, von *Trigonella foenum-graecum,* welcher Saponine enthält;
- die Verbindung mit antioxidativen Eigenschaften.

4. Futtermittelzusatzstoff nach einem der Ansprüche 1 bis 3, **dadurch gekennzeichnet, dass** die Verbindung mit antioxidativen Eigenschaften eine antioxidative Aktivität aufweist, die größer als oder gleich 2.500 µmol Trolox/g ist, wobei die antioxidative Aktivität durch das in der Publikation mit dem Titel *"DPPH antioxidant assay revisited"* von Om P. Sharma und Tej K. Bhat, Food Chemistry, Volume 113, Nr. 4, April 15, 2009, Seiten 1202-1205 beschriebene Verfahren gemessen wird.

5. Futtermittelzusatzstoff nach Anspruch 4, **dadurch gekennzeichnet, dass** die Verbindung mit antioxidativen Eigenschaften eine antioxidative Aktivität von zwischen 2.500 µmol Trolox/g und 25.000 µmol Trolox/g aufweist.

6. Futtermittelzusatzstoff nach einem der Ansprüche 1 bis 5, **dadurch gekennzeichnet, dass** der Futtermittelzusatzstoff zwischen 1% und 50%, vorzugsweise zwischen 1% und 25% eines Extraktes mindestens einer Pflanze umfasst, die die Verbindung mit antioxidativen Eigenschaften enthält.

7. Futtermittelzusatzstoff nach Anspruch 6, **dadurch gekennzeichnet, dass** der Extrakt einer Pflanze, die die Verbindung mit antioxidativen Eigenschaften enthält, ausgewählt ist unter den Extrakten der Pflanzen *Castanea sativa, Camellia sinensis, Rosmarinus officinalis, Vitis vinifera* und *Olea europea,* einzeln oder in deren Gemischen.

8. Futtermittelzusatzstoff nach Anspruch 7, **dadurch gekennzeichnet, dass** der Pflanzenextrakt ein Extrakt von *Castanea sativa* ist.

9. Futtermittelzusatzstoff nach einem der Ansprüche 1 bis 8, **dadurch gekennzeichnet, dass** das Verhältnis von Gewichts-% der Saponine von *Camellia oleifera* zu dem Gewichts-% der Saponine von *Trigonella foenum-graecum* zwischen 0,02: 19 und 10: 0,2, vorzugsweise zwischen 0,1: 9,5 und 4,5: 0,6 ist.

10. Futtermittelzusatzstoff nach einem der Ansprüche 1 bis 9, **dadurch gekennzeichnet, dass** das Verhältnis der Summe von Gewichts-% der Saponine von *Camellia oleifera* und von *Trigonella foenum-graecum* zu den Gewichts-% der Verbindung mit antioxidativen Eigenschaften zwischen 0,22: 25 und 29: 1, vorzugsweise zwischen 0,7: 15 und 14: 1 ist.

11. Futtermittelzusatzstoff nach einem der Ansprüche 3 bis 10, **dadurch gekennzeichnet, dass** dieser ausgedrückt in Gewichts-% relativ zu dem Gesamtgewicht des Zusatzstoffes umfasst:
- zwischen 2% und 50%, vorzugsweise zwischen 2% und 30% eines Extraktes von Samen, Blättern, Stängeln oder Wurzeln, einzeln oder in deren Gemischen, von *Camellia oleifera*;
- zwischen 20% und 95%, vorzugsweise zwischen 60% und 95% eines Extraktes von Samen, Blättern, Stängeln oder Wurzeln, einzeln oder in deren Gemischen, von *Trigonella foenum-graecum;*
- zwischen 1% und 25%, vorzugsweise zwischen 1% und 15% der Verbindung mit antioxidativen Eigenschaften oder zwischen 1% und 50%, vorzugsweise zwischen 1 und 25% eines Pflanzenextraktes, der die Verbindung mit antioxidativen Eigenschaften enthält;
- wahlweise zwischen 0,01% und 75%, vorzugsweise zwischen 0,01% und 50% einer Verbindung, ausgewählt unter Exzipienten wie Silika, Ton, Konservierungsmitteln, Mehl und Getreidenebenerzeugnissen.

12. Futtermittelzusatzstoff nach einem der Ansprüche 1 bis 11, **dadurch gekennzeichnet, dass** dieser weiter Saponine umfasst, ausgewählt unter den Saponinen der Pflanzen *Aesculus hippocastanum, Argania spinosa, Calendula officinalis*, *Camellia sinensis, Chenopodium quinoa, Gypsophila paniculata, Medicago sativa, Melilotus officinalis*, *Primula veris, Quillaja saponaria, Saponaria officinalis*, *Saponaria vaccaria* und *Yucca schidigera,* einzeln oder in deren Gemischen.

13. Tierfuttermittel, vorzugsweise für Geflügel, **dadurch gekennzeichnet, dass** dieses zwischen 0,01 und 1,00 kg, vorzugsweise zwischen 0,10 und 0,50 kg eines Futtermittelzusatzstoffes nach einem der Ansprüche 1 bis 12 pro Tonne Futtermittel umfasst.

14. Tierfuttermittel nach Anspruch 13, **dadurch gekennzeichnet, dass** dieses umfasst:
- zwischen 2,5 und 100 g/Tonne Futtermittel, vorzugsweise zwischen 2,5 und 25 g/Tonne Futtermittel von *Camellia oleifera* Saponinen oder zwischen 1 und 2.000 g/Tonne Futtermittel, vorzugsweise zwischen 3 und 650 g/Tonne Futtermittel eines Extraktes von *Camellia oleifera*;
- zwischen 2,5 und 100 g/Tonne Futtermittel, vorzugsweise zwischen 2,5 und 25 g/Tonne Saponin-Futtermittel von *Trigonella foenum-graecum* oder zwischen 25 und 10.000 g/Tonne Futtermittel, vorzugsweise zwischen 25 und 500 g/Tonne Futtermittel eines Extraktes von *Trigonella foenum-graecum;*
- zwischen 1 und 25 g/Tonne Futtermittel, vorzugsweise zwischen 1 und 15 g/Tonne Futtermittel der Verbindung mit antioxidativen Eigenschaften oder zwischen 1 und 100 g/Tonne Futtermittel, vorzugsweise zwischen 1 und 50 g/Tonne Futtermittel eines Extraktes einer Pflanze, die die Verbindung mit antioxidativen Eigenschaften enthält.

15. Futtermittelzusatzstoff nach einem der Ansprüche 1 bis 12 oder Futtermittel nach Anspruch 13 oder 14 für die präventive oder kurative Behandlung von Kokzidiose, vorzugsweise Geflügel-Kokzidiose.

## Claims

1. Food additive for animals, in particular for poultry, **characterized in that** it comprises, in weight percentage expressed relative to the total weight of said additive, at least:
- between 0,02% and 10%, preferably between 0,1% and 4,5%, saponins from the *Camellia oleifera* plant,
- between 0,2% and 19%, preferably between 0,6% and 9,5%, saponins from the *Trigonella foenum-graecum* plant, and
- between 1% and 25%, preferably between 1% and 15%, of a compound having antioxidant properties.

2. Food additive according to claim 1, **characterized in that** the food additive comprises:
- seeds, leaves, stems or roots, taken alone or in mixtures thereof, of *Camellia oleifera* which contain saponins;
- seeds, leaves, stems or roots, taken alone or in mixtures thereof, of *Trigonella foenum-graecum* which contain saponins;
- the compound having antioxidant properties.

3. Food additive according to Claim 1, **characterized in that** the food additive comprises:
- an extract of seeds, leaves, stems or roots, taken alone or in mixtures thereof, of *Camellia oleifera* which contains saponins;
- an extract of seeds, leaves, stems or roots, taken alone or in mixtures thereof, of *Trigonella foenum-graecum* which contains saponins;
- the compound having antioxidant properties.

4. Food additive according to any one of claims 1 to 3, **characterized in that** the compound having antioxidant properties has an antioxidant activity greater than or equal to 2,500 µmol trolox/g, said antioxidant activity being measured by the method described in the publication titled "DPPH antioxidant assay revisited" by Om P. Sharma and Tej K. Bhat, Food Chemistry, Volume 113, No. 4, April 15, 2009, pages 1202-1205.

5. Food additive according to claim 4, **characterized in that** the compound having antioxidant properties has an antioxidant activity of between 2500 µmol trolox/g and 25 000 µmol trolox/g.

6. Food additive according to any one of claims 1 to 5, **characterized in that** the food additive comprises between 1% and 50%, preferably between 1% and 25%, of an extract of at least one plant which contains said compound having antioxidant properties.

7. Food additive according to claim 6, **characterized in that** the extract of a plant which contains the said compound having antioxidant properties is chosen from the extracts of the plants of *Castanea sativa, Camellia sinensis, Rosmarinus officinalis, Vitis vinifera* and *Olea europea*, taken alone or in mixtures thereof.

8. Food additive according to claim 7, **characterized in that** the plant extract is an extract of *Castanea sativa.*

9. Food additive according to any one of claims 1 to 8, **characterized in that** the ratio of the weight percentage of saponins from *Camellia oleifera* to the weight percentage of saponins from *Trigonella foenum-graecum* is between 0,02: 19 and 10: 0,2, preferably between 0,1: 9,5 and 4,5: 0,6.

10. Food additive according to any one of claims 1 to 9, **characterized in that** the ratio of the sum of the weight percentages of the saponins of *Camellia oleifera* and of *Trigonella foenum-graecum* to the weight percentage of the compound having antioxidant properties is between 0,22: 25 and 29: 1, preferably between 0,7: 15 and 14: 1.

11. Food additive according to any one of claims 3 to 10, **characterized in that** it comprises in weight percentages expressed relative to the total weight of said additive:
- between 2% and 50%, preferably between 2% and 30%, of an extract of seeds, leaves, stems or roots, taken alone or in mixtures thereof, of *Camellia oleifera*;
- between 20% and 95%, preferably between 60% and 95%, of an extract of seeds, leaves, stems or roots, taken alone or as mixtures thereof, of *Trigonella foenum-graecum;*
- between 1% and 25%, preferably between 1% and 15%, of the compound having antioxidant properties or between 1% and 50%, preferably between 1 and 25%, of a plant extract which contains said compound having antioxidant properties;
- optionally between 0,01% and 75%, preferably between 0,01% and 50%, of a compound chosen from excipients such as silica, clay, preservatives, flour and cereal byproducts.

12. Food additive according to any one of claims 1 to 11, **characterized in that** it further comprises saponins chosen from the saponins of the plants *Aesculus hippocastanum, Argania spinosa, Calendula officinalis, Camellia sinensis, Chenopodium quinoa, Gypsophila paniculata, Medicago sativa, Melilotus officinalis, Primula veris, Quillaja saponaria, Saponaria officinalis, Saponaria vaccaria and Yucca schidigera,* taken alone or in a mixtures thereof.

13. Animal feed, preferably poultry, **characterized in that** it comprises between 0,01 and 1,00 kg, preferably between 0,10 and 0,50 kg, of feed additive according to any one of claims 1 to 12, per ton of feed.

14. Animal feed according to claim 13, **characterized in that** it comprises:
- between 2,5 and 100 g/tonne of feed, preferably between 2,5 and 25 g/tonne of feed from Camellia oleifera saponins or between 1 and 2000 g/tonne of feed, preferably between 3 and 650 g/tonne of feed, of an extract of *Camellia oleifera*;
- between 2,5 and 100 g/tonne of feed, preferably between 2,5 and 25 g/tonne of saponin feed from *Trigonella foenum-graecum* or between 25 and 10000 g/tonne of feed, preferably between 25 and 500 g/tonne of feed, of an extract of *Trigonella foenum-graecum;*
- between 1 and 25 g/tonne of feed, preferably between 1 and 15 g/tonne of feed, of the compound having antioxidant properties or between 1 and 100 g/tonne of feed, preferably between 1 and 50 g/tonne of feed, from an extract of a plant which contains said compound having antioxidant properties.

15. Food additive according to any one of claims 1 to 12 or food according to claim 13 or 14 for the preventive or curative treatment of coccidiosis, preferably coccidiosis of poultry.
